# EUROPÄISCHE PATENTSCHRIFT

(11) **EP 3 308 738 B1**
(45) Veröffentlichungstag und Bekanntmachung des Hinweises auf die Patenterteilung: **22.04.2020**
(21) Anmeldenummer: 16193819.6
(22) Anmeldetag: 13.10.2016
(51) Int. Cl.: A61C 13/00, A61C 9/00, A61B 5/00

(54) **VERFAHREN ZUM BEREINIGEN VON VIRTUELLEN REPRÄSENTATIONEN VON OBJEKTEN**
METHOD FOR CLEANING UP VIRTUAL REPRESENTATION OF OBJECTS
PROCÉDÉ D'APUREMENT DE REPRÉSENTATIONS VIRTUELLES D'OBJETS

(43) Veröffentlichungstag der Anmeldung: 18.04.2018
(73) Patentinhaber: a.tron3d GmbH, 9020 Klagenfurt am Wörthersee (AT)
(72) Erfinder: Jesenko, Jürgen, 9584 Finkenstein (AT); Kelz, Engelbert, 9020 Klagenfurt (AT)
(74) Vertreter: Beer & Partner Patentanwälte KG

(56) Entgegenhaltungen:
- WO-A1-2013/010910
- US-A1- 2002 037 489
- US-A1- 2015 024 337
- US-A1- 2016 070 821

## Beschreibung

Die Erfindung betrifft ein Verfahren zum Bereinigen, insbesondere zum Entfernen unerwünschter Daten, einer optisch erfassten, virtuellen Repräsentation von Zähnen und intraoralen Strukturen wobei die Repräsentation wenigstens zwei, bevorzugt wenigstens drei, aufeinander folgende Zähne darstellt.

In diesem Zusammenhang wird die US 2002/037489 A1 genannt.

Insbesondere im Bereich dentaler Behandlungen sind viele Systeme zum optischen Erfassen der dreidimensionalen Geometrie von Objekten bekannt. Diese finden beispielsweise Anwendung beim Erstellen von Prothesen, Kronen, Inlays oder dergleichen, dienen der Unterstützung bei der Überwachung von kieferorthopädischen Behandlungen und/oder helfen ganz allgemein bei der Beobachtung bzw. Erfassung von intraoralen Strukturen. Der große Vorteil derartiger optischer Systeme liegt zum einen darin, dass sie weder invasiv, noch unangenehm sind, wie beispielsweise der in der herkömmlichen Zahnmedizin häufig verwendete Zahnabdruck, und dass sie auch keine potentielle Gefährdung des Patienten darstellen, wie es beispielsweise bei strahlungsbasierten Methoden, wie dem Röntgen, der Fall sein kann. Zum anderen liegen die Daten nach dem Erfassen in elektronischer Form vor und lassen sich einfach speichern, beispielsweise für spätere Vergleiche, oder auch übermitteln, beispielsweise von einem Zahnarzt zu einem zahntechnischen Labor.

Ein Problem, das sich bei optischen Verfahren zum Erfassen der dreidimensionalen Geometrie von Objekten, insbesondere Zähnen, immer wieder stellt, ist, dass im Mundraum vorhandene Weichteile, wie die Innenseite der Wangen oder die Zunge, unbeabsichtigt erfasst werden. Eine spätere Korrektur solcher fehlerhaften Aufnahmen gestaltet sich üblicherweise schwierig, da auch bei Systemen, die mehrere Aufnahmen des selben Bereiches vorsehen, die fehlerhaften Aufnahmen in die erfasste bzw. berechnete Geometrie mit einfließen und diese verfälschen. Weiters stellen versehentlich aufgenommene Oberflächen ein unnötiges zusätzliches Datenvolumen dar, das unter Umständen verschiedene Prozesse, wie beispielsweise die Visualisierung der erfassten Oberflächengeometrie, verlangsamen kann.

Die bisher im Stand der Technik unternommenen Lösungsansätze zu diesem Problem folgen vor allem zwei grundlegenden Strategien. Bei der einen Strategie werden die fehlerhaft erfassten Oberflächen als solche identifiziert und entfernt. Ein Beispiel für diese erste Herangehensweise zeigt WO 2013/010910 A1. Bei der zweiten Strategie werden Leerräume definiert bzw. identifiziert, in denen sich keine Oberflächen befinden können, und Oberflächen, die in der Folge als in diesen Leeräumen befindlich gemessen werden, werden entweder durch das System entfernt, wenn das Identifizieren nach dem Messen erfolgt, oder von vorn herein ignoriert. Ein Beispiel für diese Herangehensweise wird in EP 2 775 256 A1 gezeigt.

Beiden Systemen gemeinsam ist, dass während oder nach dem Scannen aktiv entweder fälschlicherweise erfasste Oberflächen oder Leerräume als Fehler erfasst bzw. erkannt werden müssen, was zum einen Rechenressourcen benötigt und zum anderen anfällig für Fehler ist.

Der Erfindung liegt daher die Aufgabe zu Grunde, die oben beschriebenen Nachteile zu überwinden und ein vereinfachtes Verfahren zur Bereinigung unerwünschter Oberflächenbereiche zur Verfügung zu stellen. Bevorzugt soll dieses auch unabhängig von einer jeweils zum Zeitpunkt des Bereinigens erfassten Oberfläche erfolgen können. Das heißt, auch ohne dass der Fehler zu einer wenigstens teilweise "fertigen" Oberfläche in Bezug gebracht werden kann.

Gelöst wird diese Aufgabe erfindungsgemäß durch ein Verfahren der eingangs genannten Art, wobei das Verfahren die folgenden Schritte beinhaltet:
a. Definieren einer Erstreckungslinie der Repräsentation, die im Wesentlichen einem Kieferbogen mit den wenigstens zwei aufeinanderfolgenden Zähnen folgt,
   a1. Unterteilen der Erstreckungslinie in Punkte für das Erzeugen von Projektionsebenen, wobei die Punkte bevorzugt äquidistant verteilt sind,
b. Erzeugen einer Projektionsebene an einem der Punkte der Erstreckungslinie, wobei die Erstreckungslinie in diesem Punkt normal zu der erzeugten Projektionsebene liegt,
c. Projizieren aller bekannten Raumpunkte der Repräsentation aus einem Bereich an der Projektionsebene auf die Projektionsebene, wobei der Bereich einer Scheibe oder einem Keil vor und/oder hinter der Projektionsebene entspricht, wobei zu jedem projizierten Punkt wenigstens ein korrespondierender Raumpunkt hinterlegt wird,
d. Erzeugen einer zweidimensionalen Kurve auf der Projektionsebene aus den projizierten Punkten,
e. Bestimmen von Maxima, Minima und einer Mitte der Kurve,
f. Identifizieren von projizierten Punkten der Kurve, die von der Mitte der Kurve aus betrachtet außerhalb der Minima oder Maxima liegen,
g. Entfernen der Raumpunkte, welche zu den in Schritt f. identifizierten Punkten korrespondieren,
h. gegebenenfalls Wiederholen ab Schritt b. für einen weiteren Punkt der Erstreckungslinie.

Die Erstreckungslinie, welche in Schritt a) definiert wird, folgt dabei im Wesentlichen dem Kieferbogen. Mögliche Wege, um verschieden genaue Erstreckungslinien zu erzeugen, werden in späteren Abschnitten erläutert.

Die normal zur Erstreckungslinie erzeugte Ebene in einem Punkt der Erstreckungslinie in Schritt b) kann auch als Schnitt durch die Repräsentation betrachtet werden.

Das Projizieren aus Schritt c) zeigt folglich im Wesentlichen das Profil der Repräsentation im Schnitt bzw. in der Ebene von Schritt b). Der Bereich kann dabei verschieden gewählt werden, wie weiter unten erläutert wird.

In Schritt d) wird aus den projizierten Punkten von Schritt c) eine zweidimensionale Kurve erzeugt. Dies kann verschiedene Unterschritte, beispielsweise zum Glätten der Kurve oder zum Schließen von Lücken, beinhalten. Auf einige mögliche Zwischenschritte von Schritt d) wird ebenfalls weiter unten eingegangen.

In Schritt e) werden dann die Maxima und Minima sowie eine Mitte der Kurve bestimmt. Abhängig davon, ob es sich bei dem Objekt im Bereich der Ebene, respektive des Schnittes, um einen Backen- oder Schneidezahn handelt und ob sich dieser im Ober- oder Unterkiefer befindet, wird die Mitte etwa im Bereich von einem oder zwei größten Maxima oder Minima der Kurve liegen. Diese Mitte liegt dann im Wesentlichen in der Mitte des Zahnes und die Minima bzw. Maxima liegen auf den Spitzen der Zähne. Die weiter weg von der Mitte liegenden Minima bzw. Maxima entsprechen folglich üblicherweise einem Übergang zwischen Zahnfleisch und übrigem Weichgewebe, wie beispielsweise der Zunge oder der Wangeninnenseite. Lässt sich auf diese Art keine Mitte definieren, kann stattdessen als Mitte das arithmetische Mittel zwischen den beiden Endpunkten der Kurve definiert werden.

Identifiziert man, wie in Schritt f) vorgesehen, alle Punkte, die von der Mitte aus betrachtet außerhalb der äußeren Maxima bzw. Minima liegen, identifiziert man automatisch auch die unerwünschten Bereiche, ohne dass hierfür ein aktives Erkennen dieser Strukturen erforderlich ist. Selbstverständlich kann die Grenze zum Identifizieren auch etwas außerhalb der Maxima bzw. Minima festgesetzt werden, um erwünschte Daten nicht versehentlich zu entfernen.

In Schritt g) können dann alle entsprechenden, zu den projizierten und identifizierten Punkten korrespondierenden Raumpunkte entfernt werden. In der Folge erhält man eine bereinigte Repräsentation, ohne dass dafür falsche oder korrekte Oberflächen in einem komplizierten Verfahren aktiv ermittelt werden mussten.

Dies kann dann schrittweise für beliebig viele Punkte der Erstreckungslinie erfolgen, wie in Schritt h) definiert wird. Bevorzugt sind die einzelnen Ebenen bzw. Schnitte entlang der Erstreckungslinie dabei so beabstandet, dass jeder Teil der Repräsentation in wenigstens einem (der) Bereich(e) von Schritt c) liegt und auf eine der Ebenen aus Schritt b) projiziert wurde.

Um die Einteilung rechnerisch zu vereinfachen, können die Ebenen bzw. Schnitte bevorzugt äquidistant auf der Erstreckungslinie erzeugt werden.

Weitere bevorzugte Ausführungsformen der Erfindung sind Gegenstand der übrigen Unteransprüche.

Nachstehend sind bevorzugte Ausführungsbeispiele der Erfindung anhand der Zeichnungen näher beschrieben. Es zeigt:
- Fig. 1: eine Methode zur rechnerischen Vereinfachung einer dreidimensionalen Repräsentation, insbesondere einer TSDF,
- Fig. 2: eine Methode zum Bestimmen von Hauptachsen,
- Fig. 3: beispielhaft ein erfindungsgemäßes Verfahren,
- Fig. 4: eine Methode zum Bestimmen einer Kurve,
- Fig. 5: eine Methode zum Bestimmen der Punkte der Kurve,
- Fig. 6: eine Methode zum Glätten der Kurve,
- Fig. 7: Beispiele für Kurven,
- Fig. 8: beispielhaft die Visualisierung eines optisch erfassten Kieferabschnitts,
- Fig. 9: die beispielhafte Visualisierung von Fig. 8 mit einer Erstreckungslinie,
- Fig. 10: eine den Fig. 8 und 9 entsprechende beispielhafte Visualisierung einer bereinigten Repräsentation,
- Fig. 11: ein alternatives Verfahren, um zu einer Erstreckungslinie zu gelangen,
- Fig. 12: eine alternative Methode zum Bestimmen von Hauptachsen,
- Fig. 13: ein weiteres, alternatives Verfahren zum Erzeugen einer Erstreckungslinie,
- Fig. 14: eine stark vereinfachte Darstellung der Schritte 111 bis 114 von Fig. 11,
- Fig. 15: eine stark vereinfachte Darstellung des Schrittes 115 in Fig. 11,
- Fig. 16: eine stark vereinfachte Darstellung der Schritte 116 bis 118 in Fig. 11 und
- Fig. 17: eine stark vereinfachte Darstellung des Schrittes 119 in Fig. 11 mit einer zusätzlichen, optionalen Erweiterung des Verfahrens von Fig. 11.

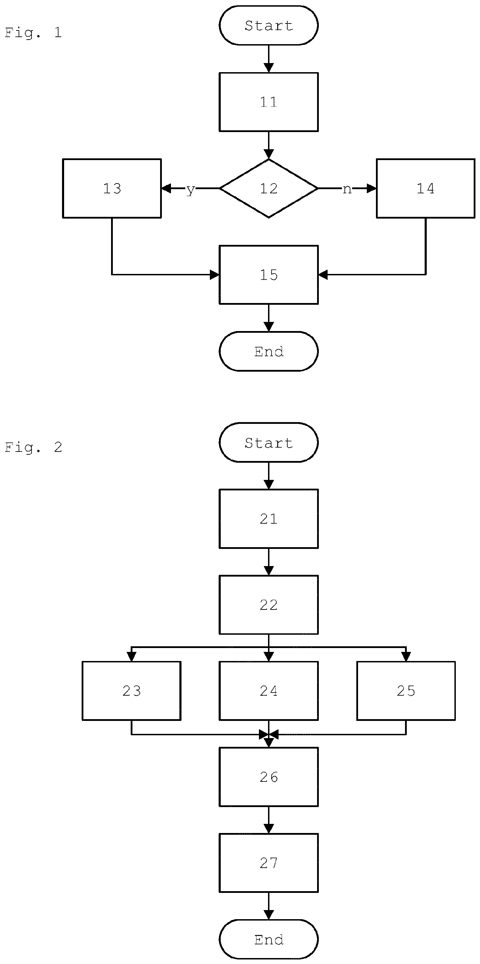

Fig. 1 zeigt eine Methode zur rechnerischen Vereinfachung einer dreidimensionalen Repräsentation, insbesondere einer TSDF. Dabei wird ein zu bereinigendes Modell zunächst in gröbere Abschnitte unterteilt. Hierfür werden jeweils Gruppen von Voxeln zu sogenannten Bricks zusammengefasst. Ein Brick ist dabei bevorzugt ein Würfel aus n³ Voxeln, bevorzugt 8³ Voxeln. Das Unterteilen der Repräsentation erfolgt in Schritt 11.

Dann erfolgt für jedes Brick die Abfrage, ob die Voxel des Bricks Oberflächeninformationen enthalten (Schritt 12).

Wird festgestellt, dass wenigstens ein Voxel des Bricks eine Oberfläche enthält, wird ein Mittelpunkt des Bricks als ein Ortsvektor notiert. Dabei entspricht der Ortsvektor einer Verbindung von einem Ursprung eines Koordinatensystems, in dem die TSDF notiert ist, zum Mittelpunkt des Bricks (Schritt 13).

Enthält ein Brick keine Voxel, die Oberflächeninformationen enthalten, wird es beispielsweise als "leer" markiert (Schritt 14).

Dann werden alle leeren Bricks und Ortsvektoren zu einer gemeinsamen Punktwolke zusammengefasst. Dabei bleibt aber für jeden Ortsvektor hinterlegt, zu welchen Voxeln er korrespondiert (Schritt 15).

Fig. 2 zeigt eine erste mögliche Methode zum Bestimmen von Hauptachsen. Hierzu wird zunächst in einem Schritt 21 die Kovarianzmatrix einer Punktwolke bestimmt. Dabei kann es sich beispielsweise um die vereinfachte Punktwolke aus den oben beschriebenen Schritten 11 bis 15 handeln. Es ist aber auch möglich, direkt mit einer Repräsentation, die in Form einer Punktwolke vorliegt, zu arbeiten. Im darauf folgenden Schritt 22 werden die drei Eigenvektoren der Kovarianzmatrix aus Schritt 21 bestimmt. Bei dreidimensionalen Punktwolken wird die Kovarianzmatrix immer drei Eigenvektoren liefern. Sind die Eigenvektoren bestimmt, werden diese als die Achsrichtungen eines Koordinatensystems definiert. Bei einer 3x3-Kovarianzmatrix, wie sie sich aus einer dreidimensionalen Punktwolke ergibt, werden die Achsrichtungen dabei immer, wie bei einem kartesischen Koordinatensystem, orthogonal zueinander stehen. Für die z-Achse wird dabei die Richtung des kleinsten Eigenvektors gewählt (Schritt 23). Für die y-Achse wird die Richtung des größten Eigenvektors gewählt (Schritt 24). Für die x-Achse wird entsprechend der mittlere Eigenvektor gewählt (Schritt 25). Wie für den Fachmann offensichtlich, können die Schritte 23 bis 25 in beliebiger Reihenfolge oder auch, wie dargestellt, parallel ausgeführt werden. Weiters wird in einem Schritt 26 der Schwerpunkt der Punktwolke bestimmt. Dieser wird dann in einem Schritt 27 als Ursprung des Koordinatensystems festgelegt. Selbstverständlich kann das Bestimmen des Schwerpunktes unabhängig von den übrigen Schritten des in Fig. 2 gezeigten Verfahrens erfolgen (abgesehen von Schritt 27). Bei hinreichender Rechenleistung kann dieser Schritt beispielsweise auch parallel zu den übrigen Schritten erfolgen. Das so erzeugte Koordinatensystem mit seinen Hauptachsen im Bezug zur Punktwolke kann in späteren Schritten des Verfahrens vorteilhaft genutzt werden.

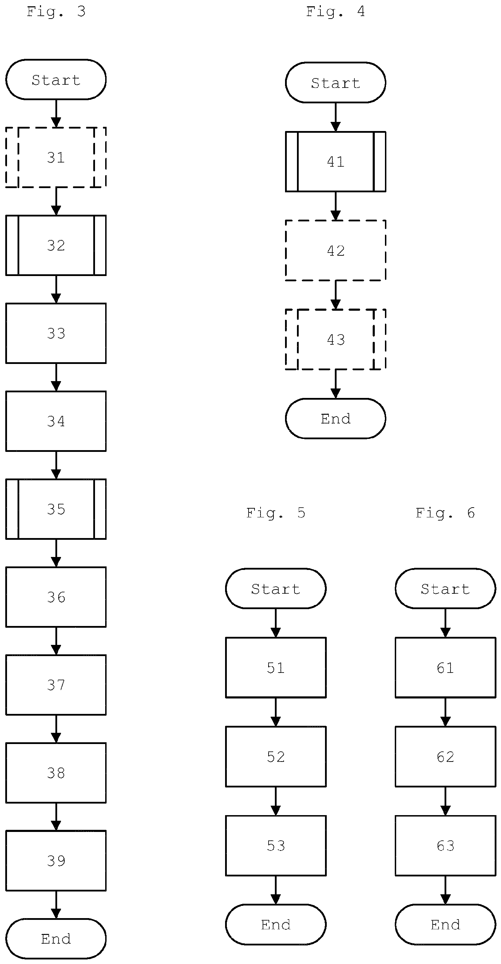

Fig. 3 zeigt beispielhaft ein erfindungsgemäßes Verfahren. Hierfür kann in Schritt 31 eine virtuelle Repräsentation zunächst, wie zu Fig. 1 beschrieben, vereinfacht werden. Dies ist für die Erfindung aber nicht zwingend erforderlich. Zum Beispiel kann dieser Schritt bei bestimmten Formaten, in denen die Repräsentation vorliegen kann, auch entfallen. Schritte, die selbstverständlich sind, wie beispielsweise das zur Verfügung stellen bzw. Laden der Repräsentation oder wie das anschließende Abspeichern, sind im Sinne der Übersichtlichkeit des dargestellten Verfahrens, nicht einzeln angeführt oder dargestellt. Entsprechende Schritte vor und nach dem eigentlichen, erfindungsgemäßen Verfahren können vom Fachmann entsprechend gewählt werden. Selbiges gilt selbstverständlich auch für die Reihenfolge der gewählten Schritte.

Im nächsten Schritt 32 wird eine Erstreckungslinie für die Repräsentation gewählt. Eine stark vereinfachte Erstreckungslinie ist eine Gerade entlang der Repräsentation.

Ein Beispiel für eine solche Gerade kann die y-Achse der in Fig. 2 bestimmten Hauptachsen sein, bzw. die längste der festgesetzten Achsen. Es können aber auch gekrümmte Erstreckungslinien bestimmt werden.

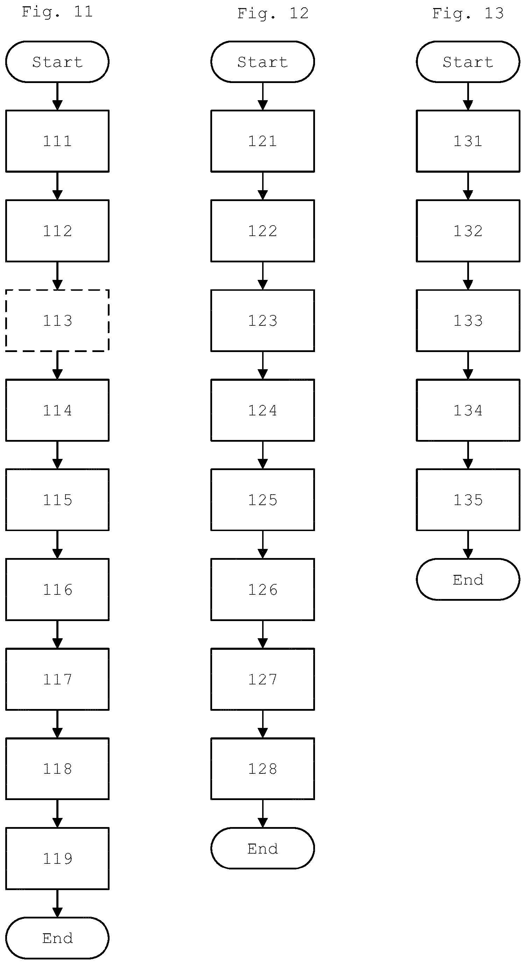

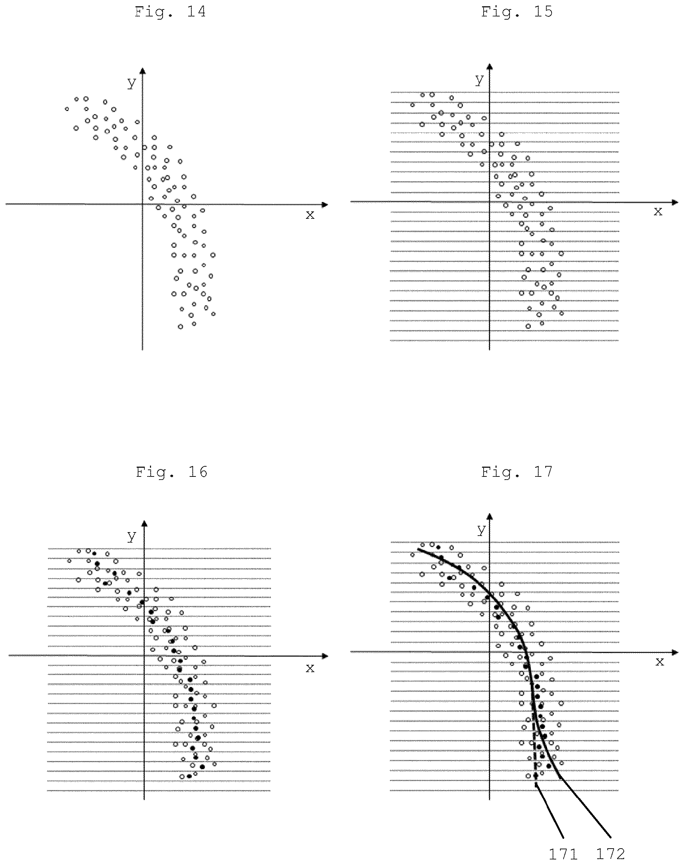

Beispiele zum möglichen Bestimmen gekrümmter Erstreckungslinien finden sich in den Fig. 11 und 13, sowie in den erläuternden Fig. 14 bis 17 zu Fig. 11. Eine alternative Methode zum Bestimmen von Hauptachsen, deren y-Achse als Erstreckungslinie verwendet werden kann, ist in Fig. 12 gezeigt.

In einem nächsten Schritt 33 werden Schnittebenen der (ggf. vereinfachten) Repräsentation erzeugt. Diese sind jedenfalls normal zur Erstreckungslinie ausgerichtet. Handelt es sich bei der Erstreckungslinie um eine Gerade, sind die Schnittebenen folglich parallel. Bevorzugt werden die Schnittebenen entlang der Erstreckungslinie äquidistant erzeugt. Wurde die Repräsentation in Schritt 31 vereinfacht, bieten sich "Scheiben" in der Dicke von jeweils einem oder zwei Bricks (beispielsweise 8 oder 16 Voxel dick) an. Ein Festsetzen der Abstände der Scheiben, kann sich auch unabhängig von der Voxelunterteilung an den tatsächlichen Maßen des repräsentierten Objektes orientieren. So kann beispielsweise ein Abstand von 2 mm gewählt werden. Eine "Scheibe" entspricht dabei dem Bereich vor und/oder hinter der Schnittebene, vorzugsweise jeweils dem Bereich vor einer Schnittebene in Richtung der Erstreckungslinie betrachtet. Es können aber beispielsweise auch mehrere "Scheiben" zusammen den Bereich bilden. Dabei können auch "Scheiben" vor und hinter der Ebene (entlang der Erstreckungslinie betrachtet) gewählt werden. Bei gekrümmten Erstreckungslinien entstehen folglich "Keile", welche die Bereiche um die Erstreckungslinien bilden können.

In einem folgenden Schritt 34 werden die Punkte in den Bereichen auf die Ebene projiziert. In sehr einfachen Anwendungen der Erfindung können beispielsweise alle Punkte innerhalb des Bereiches (die sich also in der "Scheibe" befinden) entlang von Normalen auf die Ebene abgebildet werden. Alternativ kann auch eine Projektion entlang von Normalen einer benachbarten Ebene erfolgen.

In Schritt 35 wird eine zweidimensionale Kurve auf der Ebene bestimmt. Hierzu können beispielsweise einfach alle abgebildeten Punkte verbunden werden. Eine bevorzugte und vorteilhafte Methode mit verschiedenen optionalen Varianten zum Erzeugen einer zweidimensionalen Kurve zeigt Fig. 4, welche weiter unten beschrieben wird.

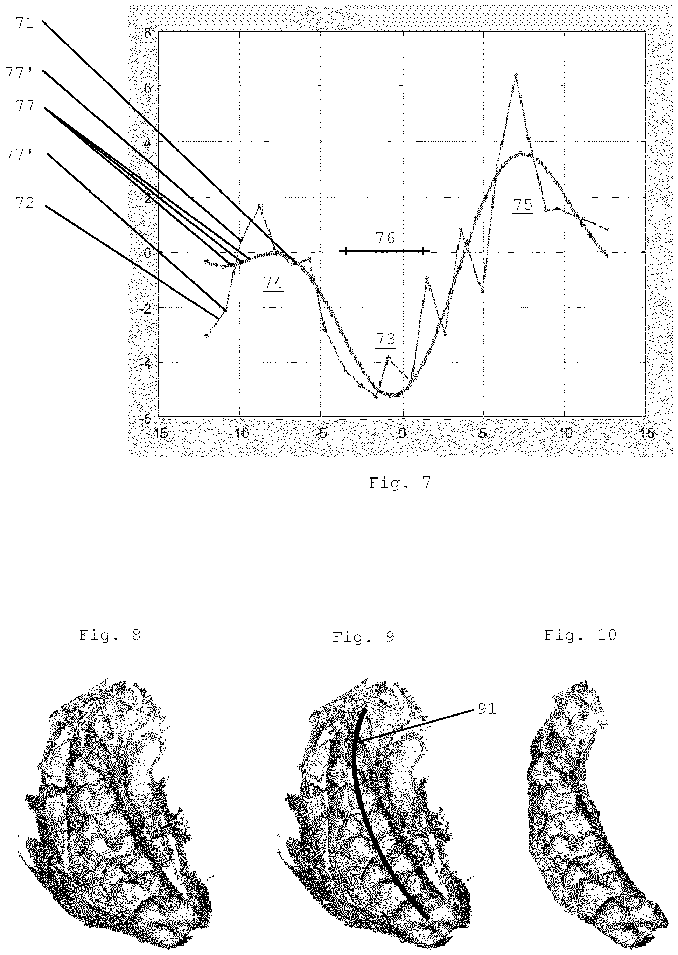

Mit der so erzeugten Kurve (siehe beispielsweise 71, 72 in Fig. 7) können dann erfindungsgemäß die Schritte 36 und 37 ausgeführt werden. Dabei werden eine Mitte (beispielsweise 73 in Fig. 7) und Minima bzw. Maxima (beispielsweise 74 und 75 in Fig. 7) links und rechts der Mitte bestimmt. Dabei ist es vorteilhaft aber nicht zwingend, wenn zuerst die Mitte der Kurve ermittelt wird.

Um bei diesem Vorgang, wie erfindungsgemäß vorgesehen, tatsächlich Kriterien zur Unterscheidung zwischen Zähnen und Artefakten, wie etwa Wangenteilen, angeben zu können, muss die Orientierung des Koordinatensystems zum (ggf. vereinfachten) Modell berücksichtigt werden, da, je nachdem, ob ein "hängender" Zahn oder ein "stehender" Zahn betrachtet wird, für die Kriterien entweder "Maxima" oder "Minima" gewählt wird.

Grundsätzlich sind alle Betrachtungen, sofern sie Minima oder Maxima betreffen, entsprechend auch jeweils umgekehrt anwendbar. Der Übersicht halber wird im Folgenden lediglich die Vorgehensweise für ein Modell beschrieben, bei dem die Zähne und umgebenden intraoralen Strukturen so orientiert sind, dass die Zahnspitzen nach unten zeigen. Alle Betrachtungen lassen sich vom Fachmann einfach auf Modelle mit nach oben weisenden Zahnspitzen übertragen.

Liegen die Zähne, wie im dargestellten Beispiel von Fig. 7, mit den Zahnkronen nach unten, gilt, dass die Schnittkurven eines frei von Fehlern bzw. unerwünschten Flächen und Artefakten aufgenommenen Zahnes im Wesentlichen U-förmig verlaufen müssen. Wurden Molaren aufgenommen, sind auch W-förmige Verläufe möglich, da die Zähne in diesem Bereich mehr als eine Spitze aufweisen. Man untersucht daher, ob es relative Maxima gibt, die links oder rechts der Mitte der Schnittkurve liegen. Maxima, die nahe an der Mitte der Kurve liegen, werden nicht berücksichtigt, da die Zahnkrone aufgrund der Fissuren auch Maxima enthalten kann. Bevorzugt bleibt daher ein Bereich nahe der Mitte (siehe 76 in Fig. 7) beim Bestimmen der relevanten Maxima bzw. Minima unberücksichtigt.

Im dann folgenden Schritt 38 werden die Punkte der Kurve markiert, welche außerhalb der festgestellten Maxima (bzw. Minima) liegen (siehe 77 und 77' in Fig. 7). Um ein unerwünschtes Verfälschen der Repräsentation zu vermeiden kann dabei in der Kurve ein "Sicherheitsabstand" zu den Maxima (siehe 74, 75 in Fig. 7) gesetzt werden.

In Schritt 39 werden dann die Raumpunkte bzw. Informationen in Voxeln der TSDF gelöscht bzw. auf "unbekannt" gesetzt, welche zu den im vorherigen Schritt markierten Punkten der Kurve korrespondieren, und somit aus der Repräsentation entfernt. Damit ist das Bereinigen abgeschlossen. Der Vorgang, Voxel als "unbekannt" bzw. "ungesehen" zu markieren, ist beispielsweise in der US 2015/0024337 A1 näher beschrieben.

Fig. 4 zeigt eine mögliche Durchführungsform für Punkt 35 aus Fig. 3. Dabei werden in Schritt 41 zunächst die projizierten Punkte bestimmt. Dafür können einfach die Punkte herangezogen werden, die projiziert wurden. Eine Weiterbildung der Erfindung, in welcher die Punkte mit einer weiterentwickelten, bevorzugten Methode ermittelt werden, zeigt Fig. 5. In Schritt 42 können den Punkten optional zusätzliche Punkte hinzugefügt werden. Dies kann durch lineare Interpolation zwischen jeweils zwei benachbarten Punkten erfolgen. Die höhere Dichte an Punkten kann dabei für verschiedene weitere Verfahren vorteilhaft sein. Ebenfalls optional kann dann die Kurve in Schritt 43 geglättet werden. Ein mögliches Verfahren für das Glätten der Kurve zeigt Fig. 6.

In dem in Fig. 5 dargestellten, bevorzugten Verfahren zum Erzeugen von Punkten der Kurve werden die Punkte, welche projiziert wurden und nun in einer zweidimensionalen Punktewolke vorliegen, in, bevorzugt vertikale, Segmente bzw. Streifen gleicher Breite unterteilt. Wurde das Modell vorher in Schritt 31 (bzw. den Schritten 11 bis 15) vereinfacht, bieten sich Streifen in der Breite von ein bis zwei Bricks an. Dann wird in einem Schritt 52 für jedes Segment jeweils ein (zweidimensionaler) Schwerpunkt ermittelt. Die in Schritt 52 ermittelten Schwerpunkte werden dann als Punkte der Kurve notiert. So können auch ungenaue Punktwolken, bei denen mehrere (einander ggf. wiedersprechende) Oberflächeninformationen vorliegen, zu Informationen vereinfacht werden, die sich leicht weiterverarbeiten lassen.

Die in Schritt 52 gewonnenen Punkte werden dann in Schritt 35 auf der Ebene aus Schritt 33 als Punkte der Kurve eingetragen.

Dabei kann es vorkommen, dass die Kurve sehr "gezackt" ist, was für verschiedene Analysen nachteilig sein kann. Darum kann die Kurve, wie bereits erwähnt, in Schritt 43 geglättet werden. Eine Möglichkeit hierfür zeigt Fig. 6. Dabei wird zunächst in einem Schritt 61 eine Fourier-Transformation auf die Kurve aus Schritt 41 bzw. 42 angewendet. Im nächsten Schritt 62 werden alle Frequenzen außer der Grundfrequenz und der ersten Harmonischen entfernt. Optional kann auch die zweite Harmonische beibehalten werden. Führt man dann in Schritt 63 die Fourier-Transformation rückwärts durch, erhält man eine sichtbar geglättete Kurve (vgl. 71 und 72 in Fig. 7), an welcher sich leicht Minima und Maxima (73,74 und 75 in Fig. 7) feststellen lassen.

Fig. 7 zeigt jeweils ein Beispiel für eine geglättete Kurve 71 und eine ungeglättete Kurve 72 mit jeweils den Punkten 77 und 77'. In beiden Kurven kann man einen Mittelbereich 76 mit einem Minimum 73 und den beiden Maxima 74 und 75 erkennen.

In einer bevorzugten, alternativen Weiterbildung der Erfindung kann nach dem Bestimmen der Mitte der Kurve auch ein gemeinsamer Abstand für alle Mitten gesetzt werden. Dieser kann insbesondere der halben bis ganzen Dicke eines Molars, insbesondere 2/3 der Dicke eines Molars, entsprechen. Hierfür kann beispielsweise eine gemessene Dicke hinzu gezogen werden. Sollten für derartige Aussagen über das zu vermessende Objekt noch zu wenige Daten vorliegen, kann aber beispielsweise auch auf statistische Daten zurückgegriffen werden, um einen entsprechenden Abstand zu wählen. Üblicherweise wird aber ein Abstand von 5 mm bis 7 mm zielführend sein.

Um bei dieser Methode jedoch kein Verfälschen der Repräsentation zu bewirken, ist es sinnvoll, die Mitten vor dem Anwenden des Abstandes zu den Mitten hinsichtlich der Wahrscheinlichkeit, dass die Mitten auf den tatsächlichen Zahnmitten liegen, zu optimieren. Dafür werden die zuvor in den Kurven auf den Ebenen bzw. Schnitten ermittelten Mitten auf eine aus der x- und der y-Achse aufgespannte Mitten-Projektionsebene projiziert. Zu diesen Punkten wird dann auf der Mitten-Projektionsebene eine Mitten-Kurve gebildet. Methoden, um diese Kurve zu bilden und in einer Erweiterung zusätzlich zu optimieren, werden zu den Fig. 11 und 17 erläutert.

Wurde die Mitten-Kurve gebildet, können die außerhalb des festzusetzenden Abstandes liegenden Bestandteile der Repräsentation entfernt werden. Hierfür ist es nicht notwendig, diese neuen Mitten in den Schnitten bzw. Ebenen anzuwenden. Viel einfacher kann der Abstand direkt im gesamten Modell (unabhängig von den Schnitten) angewendet werden. Dazu werden einfach im zuvor bestimmten oder festgesetzten Abstand zwei Parallelkurven zur Mitten-Kurve erzeugt. Diese Parallelkurven werden dann normal zur Mitten-Projektionsebene (also entlang der z-Achse) zu (Parallel-)Flächen aufgespannt. Der Bereich zwischen den Flächen wird dann in der Repräsentation belassen. Der Bereich außerhalb wird entfernt.

Die so entstandenen Begrenzungsflächen können in einer weiteren und unabhängig von der Erfindung vorteilhaften Weiterbildung des Verfahrens genutzt werden, um künftige fehlerhafte Daten beim Erfassen der Repräsentation zu vermeiden. Dazu werden die Bereiche außerhalb der Parallelflächen von vornherein gesperrt und räumliche Informationen, die innerhalb dieser Bereiche erfasst werden, werden beispielsweise beim Erzeugen der Repräsentation einfach nicht berücksichtigt.

Die Fig. 8, 9 und 10 zeigen jeweils beispielhaft eine Visualisierung der Repräsentation vor dem Bereinigen (Fig. 8), mit einer symbolisch eingezeichneten, gekrümmten Erstreckungslinie 91 (Fig. 9) und nach dem Bereinigen (Fig. 10).

Fig. 11 zeigt eine erste Methode, mit der eine gekrümmte Erstreckungslinie bestimmt werden kann. Dabei wird zunächst in Schritt 111 ein Koordinatensystem geladen. Methoden, um dieses zu erzeugen, zeigen beispielsweise die Fig. 2 und 12. Im darauf folgenden Schritt 112 wird eine Erstreckungsprojektionsebene erzeugt, die von der x- und der y-Achse des Koordinatensystems aufgespannt wird. Optional kann nun in Schritt 113 ein Erstreckungsprojektionsbereich definiert werden. Die einfachste Möglichkeit ist dabei, einfach das komplette Modell zu projizieren. In diesem Fall wird das gesamte Modell als Projektionsbereich bestimmt. Eine zweite Möglichkeit ist, nur die Hälfte des Modells abzubilden, vorzugsweise jene Hälfte, in welcher sich die Zahnkronen befinden. Der Vorteil hierbei ist, dass der Verlauf des Kieferbogens so deutlicher hervor tritt. In diesem Fall ließe sich beispielsweise die Hälfte ober- bzw. unterhalb der Erstreckungsprojektionsebene als Projektionsbereich definieren. Eine weitere Möglichkeit ist ein scheibenförmiger Projektionsbereich, wobei die Begrenzungen der Scheibe parallel zur Erstreckungsprojektionsebene liegen und vorzugsweise nur einen geringen Abstand zueinander haben. Auch hierbei liegt der Vorteil darin, dass der Verlauf des Zahnbogens besonders gut zu erkennen ist.

Nachdem der Projektionsbereich definiert wurde, werden in Schritt 114 alle Punkte des Projektionsbereiches senkrecht (also der z-Achse des Koordinatensystems folgend) auf die Erstreckungsprojektionsebene projiziert. Dadurch entsteht eine 2D-Punktwolke (symbolisch und stark schematisiert dargestellt in Fig. 14). Diese Punktwolke wird dann in Schritt 115 in gleich große Streifen unterteilt, die jeweils parallel zur x-Achse verlaufen (Fig. 15).

Innerhalb jedes Streifens werden dann in Schritt 116 der größte und der kleinste x-Wert bestimmt und in Schritt 117 das arithmetische Mittel gebildet. Aus dem arithmetischen Mittel aus Schritt 117 und der Mitte des Streifens auf der y-Achse wird dann in Schritt 118 ein jeweils einem Streifen zugeordneter, in Fig. 16 schwarz eingezeichneter Punkt für jeden Streifen bestimmt. Sollte, beispielsweise aufgrund von Lücken in der Messung und/oder Projektion, ein Streifen keine Punkte enthalten, wird dieser Streifen in den folgenden Schritten ignoriert.

Aus den Punkten von Schritt 118 kann dann in Schritt 119 eine Kurve bestimmt werden. Eine besonders geeignete und bevorzugte Methode hierfür ist die Methode der Kleinsten Quadrate. Es können aber auch andere Approximationsverfahren angewandt werden. Eine mögliche, approximierte Kurve 172, die nach dem in Fig. 11 gezeigten Verfahren entstanden ist, zeigt Fig. 17.

Weiters illustriert Fig. 17 eine optionale Erweiterung des Verfahrens von Fig. 11.

Es hat sich gezeigt, dass eine Approximation auf ein Polynom dritten Grades den vorderen Bereich (Schneidezähne) des Verlaufs eines Zahnbogens besonders gut trifft. Allerdings weicht die Kurve im hinteren Bereich (also in Richtung der Molaren) stärker vom Verlauf des Kieferbogens ab als eine einfache, gerade Erstreckungslinie. Um die Vorteile der guten Annäherung des Polynoms im vorderen Bereich zu behalten und dabei dennoch die starke Abweichung im hinteren Bereich zu vermeiden, wird in einer Erweiterung des Verfahrens von Fig. 11 eine kombinierte Erstreckungslinie gebildet. Dazu wird zunächst ein Polynom dritten Grades auf die in Fig. 11 illustrierte Weise erzeugt. Dann werden Wendepunkt und Wendetangente des Polynoms ermittelt. Als Erstreckungslinie wird dann im vorderen Bereich das Polynom festgesetzt und ab dem Wendepunkt die Wendetangente. Ein derartiger, alternativer Verlauf der Erstreckungslinie wird durch die gestrichelte Linie 171 in Fig. 17 illustriert.

Selbstverständlich ist diese Erweiterung auch auf andere Verfahren zum Ermitteln einer gekrümmten Erstreckungslinie anwendbar, bei denen ein Polynom dritten Grades ermittelt wird, um den Verlauf des Zahnbogens zu approximieren.

Fig. 12 zeigt eine alternative Methode zum Erzeugen von Hauptachsen, die in Schritt 32 erfolgen kann. Dabei werden zunächst in einem Schritt 121 die Normalvektoren der Oberfläche der Repräsentation bestimmt. Hierfür kann, wenn die Repräsentation in dieser Notation vorliegt, die TSDF herangezogen werden. Wurde die Notation, wie in Fig. 1 gezeigt, vereinfacht, wird der Normalvektor für jedes eine Oberfläche enthaltende Voxel des Bricks bestimmt und dann ein mittlerer Normalvektor erzeugt, indem alle Normalvektoren vektoriell addiert und der resultierende Vektor dann wieder auf Länge 1 gebracht wird. Der resultierende Vektor wird dann als Normalvektor des Bricks definiert.

Im darauf folgenden Schritt 122 werden die Normalvektoren aus Schritt 121 auf eine Einheitskugel projiziert (Gauß-Projektion). Als Mittelpunkt der Einheitskugel kann einfach der Ursprung des Koordinatensystems verwendet werden, in dem die Repräsentation bzw. ihre Vereinfachung notiert ist. Alternativ kann der Schwerpunkt der Repräsentation verwendet werden. Beide Varianten gleichzeitig sind beispielsweise dann abgedeckt, wenn das Koordinatensystem, in welchem die Repräsentation bzw. ihre Vereinfachung notiert ist, nach dem in Fig. 2 gezeigten Verfahren erzeugt wurde.

Die in Schritt 122 entstandene Gauß-Abbildung kann dann im folgenden Schritt 123 auf freie Flächen untersucht werden. Dabei wird davon ausgegangen, dass, selbst wenn das Modell Lücken aufweist, in denen keine Daten erfasst werden konnten, in jedem Fall keine Daten im Bereich des Kieferknochens selbst erfasst werden können. Einen größeren Bereich zu identifizieren, in dem keine Abbildung auf der Kugel vorliegt, bedeutet daher gleichzeitig den Kiefer. bzw. den "Ursprung" des repräsentierten Zahnes zu identifizieren. Wird dann in Schritt 124 eine Mitte dieses Bereiches bestimmt und dann in Schritt 125 eine Verbindung vom Mittelpunkt der Kugel zum Mittelpunkt des Bereiches gezogen, kann man davon ausgehen, dass diese Verbindung im Wesentlichen der Ausrichtung der repräsentierten Zähne entspricht. Folglich wird die in Schritt 125 erzeugte Verbindung als Richtung der z-Achse festgesetzt. Dadurch wird eine optimale Ausrichtung der Repräsentation zum Koordinatensystem bewirkt.

Eine Methode zum Bestimmen der (ungefähren) Mitte des leeren Bereiches in Schritt 124 könnte beispielsweise beinhalten, dass zunächst der Schwerpunkt aller abgebildeten Punkte auf der Gaußkugel bestimmt wird. Dieser Schwerpunkt der abgebildeten Punkte auf der Gaußkugel wird dann etwas vom Mittelpunkt versetzt und dem leeren Bereich genau gegenüber liegen. Zieht man dann eine Verbindung vom Schwerpunkt der abgebildeten Punkte auf der Gaußkugel zum Mittelpunkt hin, weist dieser automatisch in Richtung der Mitte des leeren Bereiches. Er muss dann nur noch (unter Beibehalten der Richtung) auf die Länge 1 gesetzt werden und man erhält den oben beschriebenen Vektor, welche dann in Schritt 125 als z-Achse festgesetzt wird.

Für die Bestimmung der weiteren Achsen des Koordinatensystems wird in einem Schritt 126 zunächst der größte Eigenvektor der Repräsentation bestimmt. Dieser wird in der Regel nicht orthogonal zur zuvor definierten z-Achse stehen und ist daher ungeeignet, selbst als Achse verwendet zu werden. Daher wird in Schritt 127 zunächst ein erstes Kreuzprodukt aus dem größten Eigenvektor und der z-Achse bestimmt. Die Richtung des resultierenden Vektors wird dann als die Richtung der x-Achse definiert. Zum Bilden der Richtung der y-Achse wird dann einfach in Schritt 128 das Kreuzprodukt aus der definierten z-Achse aus Schritt 125 und der definierten x-Achse aus Schritt 127 gebildet.

Alternativ kann in Schritt 128 auch das Kreuzprodukt aus der in Schritt 127 gebildeten x-Achse und dem in Schritt 126 ermittelten, größten Eigenvektor gebildet werden, um eine neue z-Achse zu ermitteln. Der größte Eigenvektor bleibt dann als y-Achse erhalten.

Wird die in Fig. 12 dargestellte Methode zum Bilden eines Koordinatensystems angewandt, so erscheinen die Zähne immer "hängend", unabhängig davon, ob es sich tatsächlich um Zähne aus dem Oberkiefer handelt oder um Zähne aus dem Unterkiefer. Abgesehen von der Frage, ob beim Bereinigen die äußeren Maxima oder Minima herangezogen werden, ist dies aber für die Funktionalität des Verfahrens unerheblich.

Die in Fig. 13 gezeigte Methode stellt eine weitere alternative und vorteilhafte Methode dar, um in Schritt 32 (Fig. 3) zu einer Erstreckungslinie zu gelangen. Dazu wird in einem Schritt 131 zunächst eine (gegebenenfalls vereinfachte) Repräsentation, die bereits über ein zentriertes und ausgerichtetes Koordinatensystem verfügt, geladen. Mögliche Wege zum Erzeugen eines solchen Koordinatensystems zeigen die Verfahren in den Fig. 2 und 12. Auch bei dem Verfahren von Fig. 13 wird dann in einem Schritt 132 eine Erstreckungsprojektionsebene zwischen der x- und der y-Achse aufgespannt. Im folgenden Schritt 133 wird die gesamte Repräsentation orthogonal auf die Erstreckungsprojektionsebene abgebildet. Auf die so entstandene zweidimensionale Punktewolke wird dann in Schritt 134 die Methode der Kleinsten Quadrate angewendet und die daraus resultierende Kurve wird notiert. Die in Schritt 134 festgesetzte Kurve wird dann in einem letzten Schritt 135 als Erstreckungslinie definiert.

Auch für das Verfahren von Fig. 13 kann die Erweiterung, bei welcher ab dem Wendepunkt die Wendetangente als Erstreckungslinie festgesetzt wird, welche zu den Fig. 11 und 17 erläutert wurde, für Kurven, die als Polynom dritten Grades angenähert wurden, angewandt werden.

Grundsätzlich kann die beschriebene Technologie sowohl nach dem Scannen als auch während des Spannens erfolgen. Sollte Letzteres gewünscht sein, kann beispielsweise ein Abbild (Clone) der Repräsentation erzeugt, parallel zum Erfassen bearbeitet und zu einem späteren Zeitpunkt mit der gerade im Erfassen befindlichen Repräsentation zusammengeführt werden. Ein hierfür geeignetes Verfahren zeigt beispielweise das österreichische Gebrauchsmuster mit der Anmeldenummer GM 50210/2016.

### Beschriftung der Figuren:

Fig. 1
   - 11: Zerlegen des Modells in Bricks
   - 12: Voxel in Brick enthalten Oberflächeninformation?
   - 13: Bestimmen eines gemeinsamen Ortsvektors für alle Voxel des Bricks
   - 14: Brick als "Leer" markieren
   - 15: Zusammenführen aller Ortsvektoren und leeren Bricks zu einer vereinfachten Punktwolke
Fig. 2
   - 21: Bestimmen der Kovarianzmatrix der Punktwolke aus den Schritten 11 bis 15 (Fig. 1) bzw. Schritt 31 (Fig. 3)
   - 22: Bestimmen der drei Eigenvektoren der Kovarianzmatrix von Schritt 21
   - 23: Festsetzen des kleinsten Eigenvektors als Richtung der z-Achse
   - 24: Festsetzen des größten Eigenvektors als Richtung der y-Achse
   - 25: Festsetzen des mittleren Eigenvektors als Richtung der x-Achse
   - 26: Bestimmen des Schwerpunktes der Punktewolke
   - 27: Festsetzen des Schwerpunktes als Ursprung eines Koordinatensystems mit den Achsen aus den Schritten 23, 24 und 25
Fig. 3
   - 31: (optional) Vereinfachen des Modells (siehe Schritte 11 bis 15 von Fig. 1)
   - 32: Bestimmen einer Erstreckungslinie (beispielsweise die y-Achse der Hauptachsen, siehe Schritte 21 bis 27 von Fig. 2 oder Schritte 121 bis 128 von Fig. 12; oder eine gekrümmte Erstreckungslinie, siehe Schritte 111 bis 119 von Fig. 11 oder Schritte 131 bis 135 von Fig. 13)
   - 33: Erzeugen von Schnittebenen normal zur Erstreckungslinie
   - 34: Projizieren auf die Ebenen
   - 35: Bestimmen der zweidimensionalen Kurve (siehe Schritte 41 bis 43 von Fig. 4)
   - 36: Bestimmen einer Mitte der Kurve aus Schritt 35
   - 37: Bestimmen der Minima bzw. Maxima links und rechts der Mitte
   - 38: Markieren der Punkte der Kurve außerhalb der äußeren Minima bzw. Maxima von Schritt 37
   - 39: Entfernen der zu den (in Schritt 38) markierten Punkten der Kurve gehörigen Raumpunkte bzw. Voxel
Fig. 4
   - 41: Bestimmen der projizierten Punkte (siehe Schritte 51 bis 53 von Fig. 5)
   - 42: (optional) Hinzufügen von weiteren Punkten durch Interpolation zwischen jeweils zwei benachbarten Punkten
   - 43: (optional) Glätten (siehe Schritte 61 bis 63 von Fig. 6)
Fig. 5
   - 51: Festsetzen von Streifen
   - 52: Bestimmen der Schwerpunkte der Streifen
   - 53: Eintragen der Schwerpunkte als Punkte der Kurve
Fig. 6
   - 61: Durchführen einer Fouriertransformation auf die Punkte aus Schritt 41
   - 62: Entfernen der hohen Frequenzen (alles außer Grundfrequenz und erster harmonischer Frequenz auf Null setzen)
   - 63: Durchführen einer inversen Fouriertransformation
Fig. 7
   - 71: eine geglättete Kurve (siehe auch Schritte 61 bis 63 von Fig. 6)
   - 72: eine ungeglättete Kurve (siehe auch Schritte 51 bis 53 von Fig. 5)
   - 73: eine Mitte der Kurve
   - 74: ein erstes Maximum (links) der Kurve
   - 75: ein zweites Maximum (rechts) der Kurve
   - 76: ein Bereich um die Mitte
Fig. 9
   - 91: eine symbolische, gekrümmte Erstreckungslinie
Fig. 11
   - 111: Laden der Repräsentation mit Koordinatensystem (siehe Fig. 2 oder Fig. 12)
   - 112: Aufspannen einer Erstreckungsprojektionsebene, die von der x- und y- Achse des Koordinatensystems aufgespannt wird
   - 113: (optional) definieren eines Erstreckungsprojektionsbereiches
   - 114: Projizieren der Punkte des Erstreckungsprojektionsbereiches auf die Erstreckungsprojektionsebene und Erzeugen einer 2D-Punktewolke
   - 115: Zerlegen der 2D-Punktewolke aus Schritt 114 in Streifen
   - 116: Bestimmen des jeweils größten und kleinsten x-Wertes je Streifen aus Schritt 115
   - 117: Bilden des arithmetischen Mittels der beiden Werte aus Schritt 116 für jeden Streifen aus Schritt 115
   - 118: Bilden eines Punktes je Streifen aus Schritt 115 mit dem arithmetischen Mittel aus Schritt 117 und der Mitte des Streifens auf der y-Achse
   - 119: Aus den Punkten von Schritt 118 eine Kurve bilden, die als Erstreckungslinie definiert wird.
Fig. 12
   - 121: Zur Verfügung stellen einer vektoriellen Repräsentation, bspw. aus dem Verfahren von Fig. 1
   - 122: Projizieren der Vektoren der Repräsentation von Schritt 121 auf eine Einheitskugel (Gauß-Abbildung)
   - 123: Überprüfen der Kugel auf einen größeren freien Bereich (ohne projizierte Vektoren),
   - 124: Bestimmen eines Mittelpunktes des Bereiches aus Schritt 123
   - 125: Festsetzen der Richtung der z-Achse des Koordinatensystems als Richtung der Verbindung vom Mittelpunkt der Einheitskugel zum Mittelpunkt des Bereiches aus Schritt 124
   - 126: Bestimmen des größten Eigenvektors der Repräsentation
   - 127: Bilden eines ersten Kreuzproduktes der z-Achse aus Schritt 125 und des größten Eigenvektors aus Schritt 127 und Festsetzen des ersten Kreuzproduktes als x-Achse
   - 128: Bilden eines zweiten Kreuzproduktes aus z-Achse und x-Achse und Festsetzen des zweiten Kreuzproduktes als y-Achse
Fig. 13
   - 131: Laden der Repräsentation mit Koordinatensystem (siehe Fig. 2 oder Fig. 12)
   - 132: Aufspannen einer Erstreckungsprojektionsebene zwischen der x- und y- Achse des Koordinatensystems
   - 133: Orthogonales Abbilden aller Punkte der (ggf. vereinfachten) Repräsentation auf der Erstreckungsprojektionsebene
   - 134: Anwenden der Methode der kleinsten Quadrate auf die Abbildung aus Schritt 133 und Notieren der resultierenden Kurve
   - 135: Festsetzen der Kurve aus Schritt 134 als Erstreckungslinie
Fig. 17
   - 171: als Polynom dritten Grades angenäherte Erstreckungslinie
   - 172: alternativer Verlauf der Erstreckungslinie ab dem Wendepunkt entsprechend der Wendetangente des Polynoms 171

## Patentansprüche

1. Verfahren zum Bereinigen, insbesondere zum Entfernen unerwünschter Daten einer optisch erfassten, virtuellen Repräsentation von Zähnen und intraoralen Strukturen, wobei die Repräsentation wenigstens zwei, bevorzugt wenigstens drei, aufeinander folgende Zähne darstellt, wobei das Verfahren die folgenden Schritte beinhaltet:
a. Definieren einer Erstreckungslinie der Repräsentation, die im Wesentlichen einem Kieferbogen mit den wenigstens zwei aufeinanderfolgenden Zähnen folgt,
a1. Unterteilen der Erstreckungslinie in Punkte für das Erzeugen von Projektionsebenen, wobei die Punkte bevorzugt äquidistant verteilt sind,
b. Erzeugen einer Projektionsebene an einem der Punkte der Erstreckungslinie, wobei die Erstreckungslinie in diesem Punkt normal zu der erzeugten Projektionsebene liegt,
c. Projizieren aller bekannten Raumpunkte der Repräsentation aus einem Bereich an der Projektionsebene auf die Projektionsebene,wobei der Bereich einer Scheibe oder einem Keil vor und/oder hinter der Projektionsebene entspricht wobei zu jedem projizierten Punkt der korrespondierende Raumpunkt hinterlegt wird,
d. Erzeugen einer zweidimensionalen Kurve auf der Projektionsebene aus den projizierten Punkten,
e. Bestimmen von Maxima, Minima und einer Mitte der Kurve,
f. Identifizieren von projizierten Punkten der Kurve, die von der Mitte der Kurve aus betrachtet außerhalb der Minima oder Maxima liegen,
g. Entfernen der Raumpunkte, welche zu den projizierten Punkten korrespondieren, die in Schritt f. identifiziert wurden,
h. gegebenenfalls Wiederholen ab Schritt b. für einen weiteren Punkt der Erstreckungslinie.

2. Verfahren nach Anspruch 1, **dadurch gekennzeichnet, dass** jeweils zwei benachbarte Projektionsebenen ein Segment der Repräsentation begrenzen.

3. Verfahren nach Anspruch 2, **dadurch gekennzeichnet, dass** der Bereich in Schritt c. durch wenigstens ein an die Projektionsebene grenzendes Segment gebildet wird.

4. Verfahren nach Anspruch 3, **dadurch gekennzeichnet, dass** die Raumpunkte eines jeden Segments wenigstens einmal projiziert werden.

5. Verfahren nach einem der Ansprüche 1 bis 4, **dadurch gekennzeichnet, dass** die Projektion in Schritt c. entlang von Normalen der Projektionsebene erfolgt.

6. Verfahren nach einem der Ansprüche 1 bis 5, **dadurch gekennzeichnet, dass** das Erzeugen der Kurve in Schritt d. durch, bevorzugt äquidistante, lineare Interpolation der projizierten Punkte auf der erzeugten Projektionsebene erfolgt.

7. Verfahren nach einem der Ansprüche 1 bis 6, **dadurch gekennzeichnet, dass** die Kurve, bevorzugt durch gegebenenfalls mehrmaliges und/oder inverses Ausführen einer Fourier-Transformation, geglättet wird.

8. Verfahren nach einem der Ansprüche 1 bis 7, **dadurch gekennzeichnet, dass** das Definieren der Erstreckungslinie in Schritt a. folgende Schritte beinhaltet:
a1. Bestimmen der Eigenvektoren der Repräsentation,
a2. Definieren des längsten Eigenvektors als Richtung der Erstreckungslinie,
a3. Definieren der Länge der Repräsentation entlang des längsten Eigenvektors als Länge der Erstreckungslinie.

9. Verfahren nach einem der Ansprüche 1 bis 8, **dadurch gekennzeichnet, dass** es sich bei der virtuellen Repräsentation um eine TSDF handelt.

10. Verfahren nach einem der Ansprüche 1 bis 9, **dadurch gekennzeichnet, dass** der Schritt h. erfolgt, bis für alle Punkte zum Erzeugen von Projektionsebenen aus Schritt a1. die Schritte b. bis g. erfolgt sind.

11. Verfahren nach einem der Ansprüche 1 bis 10, **dadurch gekennzeichnet, dass** die Erstreckungslinie eine Sequenz von miteinander verbundenen Geraden ist.

## Claims

1. Method for cleaning up, in particular for removing, unwanted data of an optically recorded, virtual representation of teeth and intraoral structures, the representation showing at least two, preferably at least three, successive teeth, wherein the method comprises the following steps:
a. defining an extension line of the representation, which line substantially follows an arch of the jaw having the at least two successive teeth,
a1. dividing the extension line into points for generating projection planes, the points preferably being distributed equidistantly,
b. generating a projection plane at one of the points of the extension line, the extension line being normal to the generated projection plane at this point,
c. projecting all known spatial points of the representation from one region on the projection plane onto the projection plane, the region corresponding to a disk or a wedge in front of and/or behind the projection plane, the corresponding spatial point being stored for each projected point,
d. generating a two-dimensional curve on the projection plane from the projected points,
e. determining maxima, minima and a center of the curve,
f. identifying projected points on the curve that are outside the minima or maxima when viewed from the center of the curve,
g. removing the spatial points that correspond to the projected points that were identified in step f.,
h. repeating the process from step b. if necessary for another point on the extension line.

2. Method according to claim 1, **characterized in that** two adjacent projection planes delimit a segment of the representation.

3. Method according to claim 2, **characterized in that** the region in step c. is formed by at least one segment bordering the projection plane.

4. Method according to claim 3, **characterized in that** the spatial points of each segment are projected at least once.

5. Method according to any of claims 1 to 4, **characterized in that** the projection in step c. takes place along normals of the projection plane.

6. Method according to any of claims 1 to 5, **characterized in that** the generation of the curve in step d. takes place by preferably equidistant linear interpolation of the projected points on the generated projection plane.

7. Method according to any of claims 1 to 6, **characterized in that** the curve is smoothed, preferably by optionally repeated and/or inverse execution of a Fourier transformation.

8. Method according to any of claims 1 to 7, **characterized in that** the definition of the extension line in step a. comprises the following steps:
a1. determining the eigenvectors of the representation,
a2. defining the longest eigenvector as the direction of the extension line,
a3. defining the length of the representation along the longest eigenvector as the length of the extension line.

9. Method according to any of claims 1 to 8, **characterized in that** the virtual representation is a TSDF.

10. Method according to any of claims 1 to 9, **characterized in that** step h. is carried out until steps b. to g. have been carried out for all points for generating projection planes from step a1.

11. Method according to any of claims 1 to 10, **characterized in that** the extension line is a sequence of interconnected straight lines.

## Revendications

1. Procédé d'apurement, notamment de suppression de données indésirables d'une représentation virtuelle enregistrée optiquement de dents et de structures intra-orales, dans lequel la représentation présente au moins deux, de préférence au moins trois dents successives, le procédé comprenant les étapes suivantes :
a. définition d'une ligne d'extension de la représentation, laquelle ligne suit sensiblement un arc de mâchoire avec les au moins deux dents successives,
a1. subdivision de la ligne d'extension en points pour générer des plans de projection, lesdits points étant de préférence répartis de façon équidistante,
b. génération d'un plan de projection au niveau de l'un des points de la ligne d'extension, la ligne d'extension au niveau dudit point étant disposée normalement par rapport au plan de projection généré,
c. projection de tous les points spatiaux connus de la représentation à partir d'une zone au niveau du plan de projection sur le plan de projection, la zone correspondant à un disque ou à un coin devant et/ou derrière le plan de projection, le point spatial correspondant étant consigné pour chaque point projeté,
d. génération d'une courbe bidimensionnelle sur le plan de projection à partir des points projetés,
e. détermination des maxima, des minima et d'un centre de la courbe,
f. identification des points projetés de la courbe qui se trouvent en dehors des minima ou des maxima, vus du centre de la courbe,
g. suppression des points spatiaux correspondant aux points projetés qui ont été identifiés à l'étape f.,
h. répétition, le cas échéant, des étapes à partir de l'étape b. pour un autre point de la ligne d'extension.

2. Procédé selon la revendication 1, **caractérisé en ce que** deux plans de projection adjacents délimitent un segment de la représentation.

3. Procédé selon la revendication 2, **caractérisé en ce que** la zone à l'étape c. est formée par au moins un segment adjacent au plan de projection.

4. Procédé selon la revendication 3, **caractérisé en ce que** les points spatiaux de chaque segment sont projetés au moins une fois.

5. Procédé selon l'une des revendications 1 à 4, **caractérisé en ce que** la projection à l'étape c. est effectuée le long des normales du plan de projection.

6. Procédé selon l'une des revendications 1 à 5, **caractérisé en ce que** la génération de la courbe à l'étape d. par interpolation linéaire, de préférence équidistante, des points projetés est effectuée sur le plan de projection généré.

7. Procédé selon l'une des revendications 1 à 6, **caractérisé en ce que** la courbe est lissée, de préférence en effectuant une transformation de Fourier plusieurs fois et/ou inversement, le cas échéant.

8. Procédé selon l'une des revendications 1 à 7, **caractérisé en ce que** la définition de la ligne d'extension à l'étape a. comprend les étapes suivantes :
a1. détermination des vecteurs propres de la représentation,
a2. définition du vecteur propre le plus long comme direction de la ligne d'extension,
a3. définition de la longueur de la représentation le long du vecteur propre le plus long comme longueur de la ligne d'extension.

9. Procédé selon l'une des revendications 1 à 8, **caractérisé en ce que** la représentation virtuelle est un TSDF.

10. Procédé selon l'une des revendications 1 à 9, **caractérisé en ce que** l'étape h. est réalisée jusqu'à ce que les étapes b. à g. aient été réalisées pour tous les points pour la génération de plans de projection à partir de l'étape a1.

11. Procédé selon l'une des revendications 1 à 10, **caractérisé en ce que** la ligne d'extension est une séquence de lignes droites reliées.
